# EUROPÄISCHE PATENTSCHRIFT

(11) **EP 0 575 930 B1**
(45) Veröffentlichungstag und Bekanntmachung des Hinweises auf die Patenterteilung: **12.03.1997**
(21) Anmeldenummer: 93109860.2
(22) Anmeldetag: 21.06.1993
(51) Int. Cl.: A61K 9/20, A61K 9/16, A61K 9/28, A61K 9/50

(54) **Verfahren zur Herstellung von festen pharmazeutischen Retardformen**
Process for the preparation of solid sustained release pharmaceutical forms
Procédé pour la préparation de formes pharmaceutiques retard solides

(30) Priorität: 25.06.1992 DE 4220782
(43) Veröffentlichungstag der Anmeldung: 29.12.1993
(73) Patentinhaber: BASF Aktiengesellschaft, 67063 Ludwigshafen (DE)
(72) Erfinder: Grabowski, Sven, Dr., D-6700 Ludwigshafen (DE); Kah-Helbig, Astrid, Dr., D-6730 Neustadt (DE); Sanner, Axel, Dr., D-6710 Frankenthal (DE); Wendel, Kurt, Dr., D-6700 Ludwigshafen (DE)

(56) Entgegenhaltungen:
- EP-A- 0 142 877
- EP-A- 0 536 595
- US-E- 27 107

## Beschreibung

Die Erfindung betrifft die Herstellung von festen pharmazeutischen Retardformen unter Verwendung eines Pulvers (zur Direkttablettierung) oder einer daraus durch Anrühren mit Wasser rekonstituierten wäßrigen Dispersion eines Bindemittels, das durch Sprühtrocknung einer durch Emulsionspolymerisation erhaltenen Bindemitteldispersion in Gegenwart von 5 bis 50 Gew.-%, bezogen auf das Bindemittel, eines wasserlöslichen Sprühhilfsmittels mit einer Glastemperatur Tg von mindestens 60°C gewonnen wurde. Vorzugsweise enthält das Pulver zusätzlich ein Antiblockmittel.

Man kann redispergierbare Polymerisatpulver aus wäßrigen Polymerisat-Dispersionen durch Versprühen der Dispersionen unter Verwendung von Ein- oder Mehrstoffdüsen in einem heißen Luftstrom herstellen. Dies gelingt dann, wenn die Glastemperatur der Polymerisate hoch genug ist, d.h. über 50°C liegt. Andernfalls treten, meist schon beim Versprühen, spätestens aber beim Lagern, insbesondere unter der Einwirkung von Wärme und/oder Druck Verklebungen, das sogenannte "Blocken", ein. Hierunter leidet die Redispergierbarkeit der Polymerisatpulver.

Man hat versucht, das Blocken zu verhindern, in dem man den Polymerisaten, die verhältnismäßig niedere Glastemperaturen aufweisen, Schutzkolloide oder indifferente Stoffe zumischte. Man erhielt dann zwar Polymerisatpulver, die besser redispergiert werden konnten, doch wurden dadurch die Eigenschaften von aus diesen Mischungen hergestellten Überzügen verfälscht.

Der Einsatz von Sprühhilfsmitteln beim Sprühtrocknen ist üblich und beispielsweise beschrieben in DE 20 49 114, 31 43 071, 33 44 242 und 39 23 229. An einen Einsatz bei der Arzneimittelherstellung wurde dabei nicht gedacht. Dementsprechend wurde nicht auf die physiologische Verträglichkeit der Hilfsmittel geachtet.

Der Erfindung lag die Aufgabe Zugrunde, ein redispergierbares Dispersionspulver zur Verfügung zu stellen, aus dem sich ohne alle chemischen und physikalischen Hilfsmittel nur durch Rühren mit Wasser eine Dispersion rekonstituieren läßt, die zur sogenannten Feuchtgranulation von Pharmawirkstoffen und Hilfsstoffen zur Herstellung von Retard-Matrix-Tabletten eingesetzt werden kann. Diese sollten sich auch durch eine Direkttablettierung des Wirkstoffs (und gegebenenfalls weiterer Hilfsstoffe) mit dem redispergierbaren Dispersionspulver herstellen lassen. Ferner sollte das redispergierbare Dispersionspulver (in Form einer rekonstituierten Dispersion) auch zum Beschichten von wirkstoffhaltigen Kernen eingesetzt werden können. Schließlich sollte die rekonstituierte Dispersion mit Pigmenten und anderen üblichen galenischen Hilfsstoffen gut verträglich sein.

Der Wirkstoff wird bei der Herstellung von Retard-Matrix-Tabletten zusammen mit wasserlöslichen Hilfsstoffen in wasserunlösliche, unverdaubare (inerte) Hilfsstoffe eingebettet, die ein Gerüst ausbilden. Durch Herauslösen der löslichen Bestandteile entstehen Poren, durch die der Wirkstoff nach außen diffundiert. Als inerte Gerüstbildner werden hauptsächlich Polymere wie Polyvinylchlorid, Polyethylen, Polyamide, Silikone, Ethylzellulosen und Methacrylat-Acrylat-Copolymere eingesetzt. Das Wirkstoff-Hilfsstoffgemisch wird entweder direkt oder nach dem Feuchtgranulieren mit Bindemittellösungen verpreßt. Die Freigabekinetik des Wirkstoffs kann über den Gehalt an unlöslichem Polymer kontrolliert werden.

Wasserunlösliche Polymere werden zur Feuchtgranulation vorteilhaft in Form von Dispersionen eingesetzt, die durch Emulsionspolymerisation erhalten wurden. Emulsionspolymerisate enthalten wasserunlösliche, submikroskopische Latexteilchen und zeigen selbst bei hohem Feststoffgehalt (bis zu 40 Gew.-%) eine relativ geringe Viskosität, so daß bei üblicher Feuchtgranulation auch relativ große Mengen an retardierenden Polymersubstanzen eingearbeitet werden können. Beim Einsatz von wäßrigen Dispersionen entfallen alle Probleme, die organische Lösungsmittel wegen ihrer Feuchgefährlichkeit, Toxizität und Umweltbelastung verursachen.

Redispergierbare Dispersionspulver sind für diesen Zweck besonders wünschenswert, denn zum einen gestatten sie die Herstellung der Matrixtabletten durch Direkttablettierung des Wirkstoffs mit dem pulverförmigen Emulsionspolymerisat, zum anderen entfallen alle mit der Verwendung von Latices verbundenen Probleme wie Verkeimung, Kontamination, Koagulation durch Frost- oder Hitzeeinwirkung, Hautbildung, Sedimentation und nicht zuletzt hohe Transportkosten.

Für die Ausbildung einer zusammenhängenden Matrix sind weiche Polymerisate, d.h. solche mit einer niederen Glasübergangstemperatur Tg, vorteilhaft. Aber gerade derartige Emulsionspolymerisate bereiten, wie erwähnt, bei der Sprühtrocknung Schwierigkeiten. Die bekannten Sprühhilfsmittel entsprechen nicht pharmazeutischen Anforderungen.

Es wurde nun gefunden, daß wasserlösliche Polymere mit einer Glastemperatur von mindestens 60°C natürlicher oder synthetischer Herkunft, die bereits in der Galenik eingesetzt werden, vorzüglich als Sprühhilfsmittel geeignet sind, und daß sie darüber hinaus auch nicht das Retardierungsverhalten des resultierenden Retardsystems derart beeinträchtigen, daß z.B. keine starke Retardierung eines gut wasserlöslichen Wirkstoffs mehr möglich wäre. Ferner wurde gefunden, daß sich ebenfalls in der Galenik gebräuchliche wasserunlösliche Hilfsstoffe, überwiegend anorganischer Natur, vorzüglich als Antiblockmittel in dem durch Sprühtrocknung mit dem Sprühhilfsmittel gewonnenen Dispersionspulver einsetzen lassen.

Die Lösung der oben geschilderten, der Erfindung zugrunde liegenden Aufgabe besteht daher in dem Verfahren nach den Ansprüchen.

Die erfindungsgemäß erhältlichen pulverförmigen "Systeme" aus Emulsionspolymerisat, Sprühhilfsmittel und Antiblockmittel lassen sich zur Feuchtgranulation einsetzen, indem man die Dispersion durch Verrühren des Pulvers mit Wasser mit oder ohne einen Zusatz von Pigmenten und/oder anderen üblichen galenischen Hilfsmitteln und mit oder ohne Erwärmen rekonstituiert und auf einem pulvrigen Wirkstoff oder eine pulvrige Wirkstoff-Hilfsstoff-Mischung aufsprüht. Die pulverförmigen Systeme lassen sich aber zudem auch zur Direkttablettierung einsetzen, d.h. zu einer direkten Verpressung des Polymerpulvers mit dem Pharmawirkstoff und gegebenenfalls weiteren Hilfsstoffen.

Die Erfindung geht aus von wäßrigen Polymerisatdispersionen, die durch Emulsionspolymerisation ethylenisch ungesättigter Verbindungen mittels freie Radikale liefernder Initiatoren in Gegenwart üblicher Zusatzstoffe hergestellt wurden.

Geeignete ethylenisch ungesättigte Monomere sind z.B. (Meth-)Acrylsäureester von C₁- bis C₁₈-Alkoholen, wie Methylmethacrylat und Ethylacrylat, auch Hydroxyalkylester der (Meth-)Acrylsäure, Vinylester und Vinyllactame; ferner ungesättigte Mono- oder Dicarbonsäuren wie (Meth-)Acrylsäure, Malein-, Fumar- und Itakonsäure sowie Halbester oder Halbamide dieser Disäuren. Geeignete Monomere mit basischen Gruppen sind N-Vinylimidazol, N-Vinylimidazolin, N-Vinylimidazolidin, N-Vinylpyridin, Monoalkyl- bzw. Dialkylaminoalkylester oder Monoalkyl- oder Dialkylaminoalkylamide von ungesättigten polymerisierbaren Carbonsäuren. Ebenfalls eingesetzt werden können anionische Monomere wie Salze von Acrylamidoalkylsulfonsäuren, kationische Monomere wie Trimethylammonioethylmethacrylat-chlorid, vernetzende Monomere wie Methylol(meth)acrylamide und deren Derivate.

Die Auswahl der Monomeren bzw. der Monomergemische richtet sich einerseits nach den Erfordernissen des Überzugsverfahrens (Glastemperatur, Mindestfilmbildetemperatur), andererseits nach dem galenischen Verhalten des Überzugs (Löslichkeitsverhalten in verschiedenen Medien, Härte, Sprödigkeit oder Elastizität des Filmes und seine Permeabilität für den Wirkstoff).

Als freie Radikale bildende Polymerisations-Initiatoren können die üblichen, wie Wasserstoffperoxid, organische Peroxide und Hydroperoxide, gegebenenfalls in Kombination mit reduzierenden Verbindungen wie Ascorbinsäure, wasserlöslichen Azoverbindungen wie 2,2-Azobis(2-amidinopropan)-dihydrochlorid, ferner anorganische Peroxide wie Alkalimetall- oder Ammoniumsalze der Peroxodischwefelsäure in Mengen von etwa 0,1 bis 2 Gew.-%, bezogen auf die Gesamtmenge der Monomeren, verwendet werden.

Bei Bedarf können dem Polymerisationsgemisch weitere übliche Hilfsstoffe zugesetzt werden. Zu diesen Hilfsstoffen zählen Keimlatices, welche die Reproduzierbarkeit der Teilchengröße der Endprodukte verbessern, sowie Puffer, Komplexbildner, Dispergiermittel und Emulgatoren. Das Emulsionspolymerisat wird in der Regel in Gegenwart von anionischen, kationischen oder nichtionischen Emulgatoren oder deren verträglichen Gemischen in Form eines wäßrigen Latex mit einem Feststoffgehalt von 20 bis 70, vorzugsweise 30 bis 60 Gew.-% hergestellt.

Die Sprühtrocknung erfolgt in üblicher Weise in Sprühtürmen, wobei die zu trocknende Dispersion mit Hilfe von Zerstäubungsscheiben oder Ein- bzw. Mehrstoffdüsen eingesprüht werden kann. Die Trocknung der Dispersion wird mit heißen Gasen, z.B. Stickstoff oder Luft, durchgeführt. Bei der Gewinnung des trockenen Polymerisatpulvers aus dem Latex ist darauf zu achten, daß die Latexteilchen als solche erhalten bleiben und nicht zu Aggregaten verkleben.

Als Sprühhilfsmittel werden eine oder mehrere wasserlösliche Substanzen mit einem Phasenumwandlungspunkt zweiter Ordnung (Glasübergangstemperatur Tg) bei mindestens 60°C in Mengen von 5 bis 50 Gew.-%, vorzugsweise 10 bis 30 Gew.-%, bezogen auf das als Bindemittel eingesetzte Emulsions-Polymerisat, zugesetzt. Hierfür haben sich polymere wasserlösliche Substanzen bewährt, insbesondere solche mit hohen Polymerisationsgraden.

Zur Erzielung einer guten Redispergierbarkeit hat es sich bewährt, wenn die Summe der Menge an Schutzkolloid, das bei der Herstellung der Dispersionen verwendet wurde, und der Menge an Sprühhilfsmittel mindestens 6 Gew.-%, vorzugsweise 10 bis 30 Gew.-% beträgt. Als Obergrenze können 50, vorzugsweise 40 Gew.-% gelten.

Das erfindungsgemäß zu verwendende Sprühhilfsmittel zeichnet sich durch seine pharmazeutische (physiologische) Akzeptanz aus. Geeignet sind Hilfsstoffe, die in Arzneibuchmonographien beschrieben sind oder die schon jahrelang ohne Zwischenfälle in Gebrauch sind oder deren Anwendung lebensmittelrechtlich geregelt ist (s. z.B. "Katalog pharmazeutischer Hilfsstoffe", verfaßt von einer Arbeitsgruppe der Firmen Ciba-Geigy, Hoffmann-LaRoche und Sandoz; "Pharmazeutische Technologie" von H. Sucker, P. Fuchs und P. Speiser, Thieme Verlag, 1991, Kap. 5, sowie die dort angegebene Literatur; "Überzugsstoffe und Trennmittel", Lebensmittelchem. Gesellschaft - Fachgruppe der GDCh, Behr's Verlag 1990). Als Beispiele sind zu nennen:

Cellulosederivate wie Methylcellulose, Hydroxypropylcellulose, Hydroxyethylcellulose, Hydroxypropylmethylcellulose, Carboxymethylcellulose-Natrium (in allen Pharmakopöen beschrieben), Polyvinylpyrrolidone (aufgeführt z.B. in der USP/NF XVI), Copolymer aus N-Vinylpyrrolidon und Vinylacetat im Gewichtsverhältnis 60:40 (DAB-Monographie "Copolyvidon"), Stärkederivate (modifizierte Stärken nach dem Lebensmittel- und Bedarfsmittelgesetz) sowie Polyvinylalkohole (besonders gereinigte Qualitäten zur pharmazeutischen Technologie, mittlere Molmassen von 30 000 bis 200 000).

Zur Erhöhung der Lagerfähigkeit, d.h. um bei Pulvern mit niederer Glastemperatur ein Verbacken und Verblocken zu verhindern und somit die Redispergierbarkeit zu verbessern, wird das erhaltene Pulver mit 0 bis 50 Gew.-%, vorzugsweise 5 bis 25 Gew.-%, bezogen auf das Gesamtgewicht polymerer Bestandteile, an üblichen Antiblockmitteln versetzt. Dies erfolgt vorzugsweise, solange das Pulver noch fein verteilt, d.h. noch im Trockengas suspendiert ist. Vorteilhaft werden diese Mittel räumlich getrennt, aber gleichzeitig mit der Dispersion in die Trocknungsvorrichtung dosiert. Besonders geeignet sind Stoffe mit mittleren Teilchengrößen von 0,1 bis 50 µm.

Das Antiblockmittel muß natürlich ebenfalls pharmazeutisch akzeptabel sein (siehe oben). Als Beispiele sind zu nennen:

Kolloidales Siliciumdioxid (im NF XIII als "Colloidal Silicon Dioxide" beschrieben), Talkum, Calcium-, Magnesium- und Natriumcarbonat, Tricalciumorthophosphat, Magnesiumorthophosphat, mikrokristalline Cellulose und Magnesiumstearat. Sie sind in Pharmakopöen beschrieben und für Lebensmittel zugelassen.

### Beispiele 1 bis 6 - Sprühtrocknung

Eine wäßrige Dispersion aus einem Ethylacrylat-Methylmethacrylat-Emulsionscopolymer (2:1 mol, Tg ca. 7°C, Mindestfilmbildetemperatur nach DIN 53 787 ca. 4°C, Viskosität ca. 5 mPas) mit 30 Gew.-% Feststoff wurde mit einem Sprühhilfsmittel sprühgetrocknet und soweit nötig mit einem Antiblockmittel versetzt. Die Beispiele 1 bis 6 sind in Tabelle 1 zusammengefaßt. Alle Gewichtsprozent-Angaben beziehen sich auf das Emulsionspolymerisat.

**Tabelle 1**

| Beispiel | Sprühhilfsmittel | Antiblockmittel | Temperaturen in °C | | |
|---|---|---|---|---|---|
| | | | Eingang | Ausgang | Düse |
| 1 | Copolymer aus 60 Gew.-% VP¹+40 Gew.-% VAc² (20 Gew.-%) | Talkum (10 Gew.-%) | 90-100 | 60-70 | 10 |
| 2 | Copolymer aus 60 Gew.-% VP¹+40 Gew.-% VAc² (20 Gew.-%) | Tricalciumorthophosphat (10 Gew.-%) | 90-100 | 60-70 | 10 |
| 3 | abgebaute Stärke (20 Gew.-%)³ | Talkum (10 Gew.-%) | 90-95 | 65-68 | 10 |
| 4 | abgebaute Stärke (20 Gew.-%)³ | Tricalciumorthophosphat (10 Gew.-%) | 90-95 | 65-68 | 10 |
| 5 | Hydroxypropylmethylcellulose (20 Gew.-%)⁴ | nicht erforderlich | 90-100 | 60-70 | 25 |
| 6 | Polyvinylpyrrolidon (30 Gew.-%) | Talkum (10 Gew.-%) | 110-120 | 65-75 | 25 |

| | | | | | |
|---|---|---|---|---|---|
| ¹) N-Vinylpyrrolidon | | | | | |
| ²) Vinylacetat | | | | | |
| ³) Snowflake®01910, Fa. Cerestar, Krefeld | | | | | |
| ⁴) Pharmacoat®606, Shin-Etsu Chemical, Tokyo | | | | | |

Die erhaltenen Pulver waren nach 8 Monaten bei 25°C noch frei fließfähig und durch Verrühren mit kaltem Wasser redispergierbar.

### Beispiel 7

Eine wäßrige Dispersion aus einem Vinylacetat-Emulsionspolymer von der Viskosität ca. 5 mPas sec mit 30 Gew.-% Feststoff wurde mit 30 Gew.-%, bezogen auf das Polymerisat, Polyvinylpyrrolidon sprühgetrocknet. Eingangstemperatur 120 bis 130°C, Ausgangstemperatur 80 bis 90°C, Düsentemperatur 16°C.

### Beispiel 8 - Feuchtgranulation

Es wurden Matrixtabletten mit einer rekonstituierten wäßrigen Dispersion des gemäß Beispiel 3 erhaltenen Polymerisat-Pulvers nach folgender Formulierung hergestellt:
1. Zusammensetzung

| | | |
|---|---|---|
| I | Theophyllin | 125 g |
| | Ca-Hydrogenphosphat | 75 g |
| II | Polymer-Pulver (Beispiel 3) als 30 %ige wäßrige Dispersion | 10 g |
| | Wasser | 31 g |
| | Talkum | 1 g |
| III | Magnesiumstearat | 1 g |

Die Redispergierung des erfindungsgemäßen Polymerisat-Pulvers gelang durch einfaches Einrühren in kaltes Wasser.
Die Mischung I wurde im Wirbelschichtgranulator mit Dispersion II granuliert, mit III gemischt und auf einem Rundläufer mit einem Druck von 9,3 kN zu Tabletten verpreßt.
2. Physikalische Eigenschaften der Tabletten

| | |
|---|---|
| Gewicht | 211,82 mg |
| Härte nach Pharmakopöe (DAB) | 64 N |
| Abrieb nach Pharmakopöe (DAB) | 0,3 % |

3. Freisetzung des Theophyllins
Es wurde die Freisetzung des Theophyllins im Freisetzungsgerät gemäß US-Pharmakopöe XXII, Paddle-Methode, bei 50 Um/Min. bestimmt. Als Vergleich wurde eine identisch hergestellte Matrixtablette mit einem handelsüblichen Ethylacrylat-Methylmethacrylat-Copolymer (Eudragit NE 30 D, Röhm Pharma, 30 %ige Dispersion) verwendet.
Medium: wäßrige Pufferlösung vom pH 7,4

| Zeit | Eudragit NE 30 D | Polymer-Pulver Beispiel 3 |
|---|---|---|
| 1h | 23,9 | 26,5 |
| 2h | 40,1 | 45,4 |
| 3h | 51,0 | 59,3 |
| 5h | 65,7 | 77,7 |
| 8h | 81,1 | 95,7 |

Ergebnis: Die Freisetzung ist in beiden Fällen sehr ähnlich, d.h., die erfindungsgemäß hergestellte Retardform entspricht dem Stand der Technik.

### Beispiel 9 - Feuchtgranulation

Es wurden Matrixtabletten mit einer rekonstituierten wäßrigen Dispersion des gemäß Beispiel 7 erhaltenen Polymerisat-Pulvers nach folgender Formulierung hergestellt:
1. Zusammensetzung

| | | |
|---|---|---|
| I | Theophyllin | 125 g |
| | Lactose | 75 g |
| II | Polymer-Pulver (Beispiel 7) als 30 %ige wäßrige Dispersion | 10 g |
| | Wasser | 31 g |
| | Talkum | 1 g |
| III | Magnesiumstearat | 1 g |

Die Redispergierung des erfindungsgemäßen Polymerisat-Pulvers gelang durch einfaches Einrühren in kaltes Wasser.
Die Mischung I wurde von Hand mit Dispersion II granuliert, mit III gemischt und auf einem Rundläufer mit einem Druck von 9,3 kN zu Tabletten verpreßt.
2. Physikalische Eigenschaften der Tabletten

| | |
|---|---|
| Gewicht | 219 mg |
| Härte nach Pharmakopöe (DAB) | 73 N |
| Abrieb nach Pharmakopöe (DAB) | 0,25 % |

3. Freisetzung des Theophyllins
Die Freisetzungsbestimmung erfolgte wie in Beispiel 8.

| Zeit | Eudragit NE 30 D | Polymer-Pulver Beispiel 7 |
|---|---|---|
| 1h | 21,6 | 17,4 |
| 2h | 32,6 | 26,5 |
| 4h | 48,1 | 39,9 |
| 6h | 58,7 | 48,8 |
| 8h | 66,0 | 56,7 |

## Patentansprüche

1. Verwendung eines durch Sprühtrocknung einer wäßrigen Dispersion, die durch Emulsionspolymerisation erhalten wurde, in Gegenwart von 5 bis 50 Gew.-% eines wasserlöslichen, pharmazeutisch akzeptablen Sprühhilfsmittels mit einer Glastemperatur von mindestens 60°C und 0 bis 50 Gew.-%, stets bezogen auf die Trockenmasse, eines pharmazeutisch akzeptablen Antiblockmittels gewonnenen redispergierbaren Pulvers als Binde- oder Überzugsmittel für feste pharmazeutische Retardformen.

2. Verwendung nach Anspruch 1, dadurch gekennzeichnet, daß als Binde- oder Überzugsmittel ein sprühgetrocknetes Emulsionspolymerisat eingesetzt wird, das aus Vinylacetat besteht.

3. Verwendung nach Anspruch 1, dadurch gekennzeichnet, daß als Binde- oder Überzugsmittel ein sprühgetrocknetes Emulsionspolymerisat eingesetzt wird, das aus Ethylacrylat und Methylmethacrylat im Gewichtsverhältnis 2:1 besteht.

4. Verwendung nach Anspruch 1, dadurch gekennzeichnet, daß als Binde- oder Überzugsmittel ein sprühgetrocknetes Emulsionspolymerisat eingesetzt wird, das ein wasserlösliches N-Vinylpyrrolidon-Polymerisat enthält, das 0 bis 50 Gew.-% Vinylacetat einpolymerisiert enthält.

5. Verwendung nach Anspruch 1, dadurch gekennzeichnet, daß als Binde- oder Überzugsmittel ein sprühgetrocknetes Emulsionspolymerisat eingesetzt wird, bei dessen Sprühtrocknung ein wasserlösliches Polysaccharid als Sprühhilfsmittel eingesetzt wurde.

## Claims

1. The use of a redispersible powder which has been obtained by spray drying an aqueous dispersion, which has been obtained by emulsion polymerization, in the presence of 5 - 50% by weight of a water-soluble pharmaceutically acceptable spraying aid with a glass transition temperature of at least 60°C and 0 - 50% by weight, always based on dry matter, of a pharmaceutically acceptable anti-blocking agent as binder or coating material for solid pharmaceutical depot forms.

2. The use as claimed in claim 1, wherein a spray-dried emulsion polymer which is composed of vinyl acetate is employed as binder or coating material.

3. The use as claimed in claim 1, wherein a spray-dried emulsion polymer which is composed of ethyl acrylate and methyl methacrylate in the ratio 2:1 by weight is employed as binder or coating material.

4. The use as claimed in claim 1, wherein a spray-dried emulsion polymer which contains a water-soluble N-vinylpyrrolidone polymer which contains 0 - 50% by weight of vinyl acetate as copolymerized units is employed as binder or coating material.

5. The use as claimed in claim 1, wherein a spray-dried emulsion polymer in whose spray drying a water-soluble polysaccharide has been employed as spraying aid is employed as binder or coating material.

## Revendications

1. Utilisation, à titre de liants ou d'agents d'enrobage pour des formes pharmaceutiques retard solides, d'une poudre redispersible obtenue par séchage par pulvérisation d'une dispersion aqueuse qui fut obtenue par polymérisation en émulsion, en présence de 5 à 50% en poids d'un adjuvant de pulvérisation pharmaceutiquement acceptable et soluble dans l'eau, d'une température de transition vitreuse d'au moins 60°C et de 0 à 50% en poids, toujours par rapport à la masse sèche, d'un agent antiblocage pharmaceutiquement acceptable.

2. Utilisation suivant la revendication 1, caractérisée en ce que l'on met en oeuvre, à titre de liant ou d'agent d'enrobage, un polymère en émulsion séché par pulvérisation, qui se compose d'acétate de vinyle.

3. Utilisation suivant la revendication 1, caractérisée en ce que l'on met en oeuvre, à titre de liant ou d'agent d'enrobage, un polymère en émulsion séché par pulvérisation, qui se compose d'acrylate d'éthyle et de méthacrylate de méthyle dans le rapport pondéral de 2:1.

4. Utilisation suivant la revendication 1, caractérisée en ce que l'on met en oeuvre, à titre de liant ou d'agent d'enrobage, un polymère en émulsion séché par pulvérisation, qui contient un polymère de la N-vinylpyrrolidone soluble dans l'eau, qui contient 0 à 50% en poids d'acétate de vinyle, incorporés par polymérisation.

5. Utilisation suivant la revendication 1, caractérisée en ce que l'on met en oeuvre, à titre de liant ou d'agent d'enrobage, un polymère en émulsion séché par pulvérisation, au cours du séchage par pulvérisation duquel on a utilisé un polysaccharide soluble dans l'eau, à titre d'adjuvant de pulvérisation.
